Europäisches Patentamt

European Patent Office

Office européen des brevets

(19)

(11) Veröffentlichungsnummer : **0 317 510 B1**

(12) **EUROPÄISCHE PATENTSCHRIFT**

(45) Veröffentlichungstag der Patentschrift :
18.03.92 Patentblatt 92/12

(51) Int. Cl.$^5$ : **A61J 3/07**

(21) Anmeldenummer : **88810766.1**

(22) Anmeldetag : **08.11.88**

(54) **Kapsel und Verfahren zu ihrer Herstellung.**

(30) Priorität : **20.11.87 CH 4527/87**

(43) Veröffentlichungstag der Anmeldung :
**24.05.89 Patentblatt 89/21**

(45) Bekanntmachung des Hinweises auf die
Patenterteilung :
**18.03.92 Patentblatt 92/12**

(84) Benannte Vertragsstaaten :
**AT BE CH DE ES FR IT LI LU NL**

(56) Entgegenhaltungen :
**DE-U- 8 715 583**
**GB-A- 566 505**

(56) Entgegenhaltungen :
**GB-A- 2 057 878**
**US-A- 3 275 519**
**LIST-MULLER : "Arzneiformenlehre", Auflage
3, 1982, Seiten 356-359, Stuttgart, DE**

(73) Patentinhaber : **Greither, Peter
Hausen
CH-9533 Kirchberg (CH)**

(72) Erfinder : **Brocker, Erich, Dr.
Dietschwil
CH-9533 Kirchberg (CH)**

(74) Vertreter : **Wenger, René et al
Hepp & Partner AG Marktgasse 18
CH-9500 Wil (CH)**

## Beschreibung

Die Erfindung betrifft eine Kapsel gemäss dem Oberbegriff von Anspruch 1 sowie ein Verfahren zu ihrer Herstellung. Pharmazeutische Wirkstoffe und diätetische Produkte für die Gesundheitspflege werden heute in vermehrtem Masse in der Form von Kapseln, insbesondere von Gelatinekapseln verabreicht.

Die Kapsel kann aber auch nur als Behältnis für bestimmte Wirkstoffe dienen, die z.B. durch Uebergiessen mit heissem Wasser freigegeben werden, wie z.B. ein Inhalationsmittel. Schliesslich kann die Kapsel auch ein Genussmittel enthalten.

Ein Problem bei dieser Darreichungsform besteht unter anderem darin, dass aufgrund des molekularen Aufbaus der Kapselhülle unangenehme Gerüche des Kapselinhalts wie z.B. Knoblauch, Lebertran usw. durchdringen können. Dies ist für den Konsumenten äusserst unangenehm. Ein anderes Problem besteht darin, dass in manchen Fällen die oral verabreichten Kapseln die Wirkstoffe erst im Dünndarm freigeben sollen und dass die Kapselhülle daher magensaftresistent sein sollte. Zur Lösung dieser Probleme wurde bereits vorgeschlagen, die Gelatinekapseln mit einem im Magensaft unlöslichen Lack zu überziehen oder zur Geruchsbindung zu silikonisieren (List/Müller/Nürnberg, Arzneiformenlehre, 3. A., Stuttgart 1982, Seiten 356, 357). Gleichzeitig wird in der Fachliteratur aber von der Lackierung abgeraten, da die Ueberzüge von den elastischen Kapseln relativ leicht abblättern und rissig werden (a.a.O.). Auch vom Beschichten der Kapseln mit einem Silikonharz wird von der Fachwelt jedoch abgeraten, so dass die geschilderten Probleme bis heute noch keine befriedigende Lösung gefunden haben (Fahrig/Hofer-Herausgeber, Die Kapsel, Stuttgart 1983). Durch die GB-A-566 505 ist ein Verfahren bekannt geworden, bei dem eine Gelatinekapsel mit einer Schutzschicht versehen wird, die z.B. aus Zucker oder dergleichen besteht. Damit soll einerseits der Kapselinhalt geschützt werden und andererseits soll eine Verzögerung der Wirkstofffreisetzung erreicht werden. Ein sicherer Schutz vor einer Geruchspenetration ist bei einer noch akzeptablen Kapselgrösse bzw. Wandstärke aber auch hier nicht gewährleistet.

Es ist daher eine Aufgabe der Erfindung, eine Kapsel der eingangs genannten Art zu schaffen, die zur Erzielung einer Geruchsinternisierung mit einer geeigneten Schutzschicht versehen ist. Gleichzeitig soll die verletzliche Kapsel resistenter gegen mechanische Beanspruchungen werden, bei gleichzeitiger Verbesserung des Erscheinungsbildes und des Geschmacks. Diese Aufgabe wird erfindungsgemäss mit einer Kapsel gelöst, welche die Merkmale im Anspruch 1 aufweist. Da die Schutzschicht geruchsbindende Festpartikel aufweist, können allenfalls in die Schutzschicht eindringende Gasmoleküle absorbiert werden. Dazu eignet sich insbesondere Aktivkohle, Bentonit, Kieselerde oder Kieselgur.

Es hat sich überraschend gezeigt, dass die an sich wasserlösliche Kapselhülle ohne weiteres direkt mit einer wasserlöslichen Substanz beschichtet werden kann, ohne dass eine wasserresistente Zwischenschicht aus einem konventionellen Lack erforderlich ist. Die Schutzschicht wirkt sich insbesondere bei Weichgelatinekapseln mit flüssigem oder pastösem Inhalt sehr vorteilhaft aus. Die Kapselhülle könnte aber auch aus Hartgelatine oder Stärke (Capill-Kapsel) gefertigt sein. Beim Aufbau einer Schichtdicke von wenigstens 200 µm wird nicht nur ein zuverlässiger Schutz gegen Geruchspenetration, sondern auch ein mechanischer Schutz der leicht verletzlichen Kapselhülle erzielt. Die Schutzschicht kann als Bestandteil eine Zuckerart oder Calciumcarbonat oder Gummi Arabicum oder Calciumstearat oder Stärke oder Talcum oder Kombinationen dieser Substanzen enthalten. Die Zusammensetzung derartiger Schichten ist an sich bereits vom Ueberziehen von festen Körpern in der Zuckerwarenindustrie, aber auch in der Pharmaindustrie bei der Herstellung von Dragées bekannt.

Die Schutzschicht kann auch färbende und/oder aromatisierende Komponenten aufweisen, um das Erscheinungsbild der Kapsel zu verbessern und um die Einnahme zu erleichtern. Schliesslich wäre es auch noch denkbar, dass die Schutzschicht Komponenten enthält, welche den Kapselinhalt vor Licht, insbesondere vor UV-Licht schützen.

Je nach Anwendungsfall kann zwischen der Kapselhülle und der Schutzschicht eine Sperrschicht aus einem Lack oder aus einem Harz angeordnet sein. Die Sperrschicht ist vorzugsweise magensaftresistent. Die entsprechenden Stoffe sind von den Film- und Lacktabletten bereits bekannt. Die Sperrschicht hält auch auf flexiblen Kapselhüllen, da die äussere Schutzschicht ein Reissen oder Abblättern verhindert.

Die Erfindung betrifft auch ein Verfahren zur Herstellung der genannten Kapseln, wobei die Bestandteile der Schutzschicht in einer wässrigen Suspension oder Lösung aufbereitet werden. Anschliessend werden die Kapselhüllen bei gleichzeitiger oder nachfolgender Trocknung so lange beschichtet, bis die gewünschte Schichtdicke erreicht ist. Das Auftragen der Suspension oder der Lösung kann dabei kontinuierlich oder schichtweise erfolgen. Zum Auftragen können an sich bekannte Verfahren wie z.B. der Sprühauftrag, der Auftrag im Wirbelbettverfahren oder das Aufgiessen in Dragierkesseln eingesetzt werden.

Bei magensaftresistenten Kapseln werden die Kapselhüllen vor dem Auftragen der Schutzschicht mit einem Lack oder mit einem Harz beschichtet. Die Kapselhülle kann aber auch auf andere Weise behandelt werden, z.B. durch Behandlung mit Formaldehyd, Crosslinking der Polymeren usw.

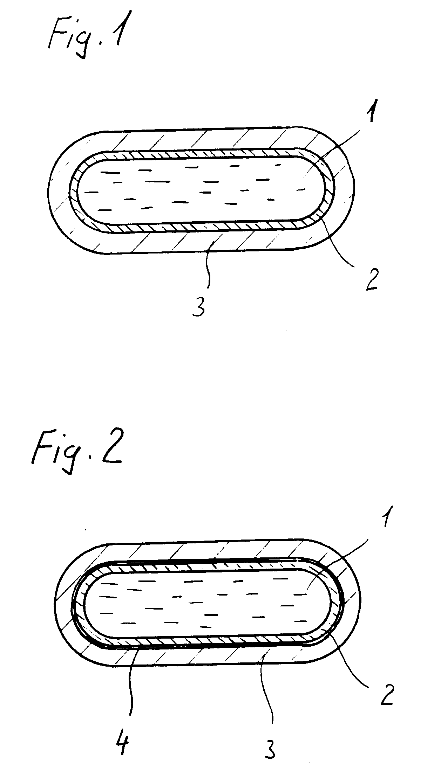

Figur 1 zeigt in vereinfachter Darstellungsweise eine Kapsel mit einer Kapselhülle 2, beispielsweise aus Weichgelatine, die eine flüssige oder pastöse Kapselfüllung 1 aufweist. Die Kapselhülle könnte aber auch aus anderen Biopolymeren wie z.B. Stärke, Gummi, Agar-Agar usw. gefertigt sein. Die Aussenseite der Kapselhülle 2 ist mit einer relativ starren Schutzschicht 3 aus einer wasserlöslichen Substanz beschichtet. Die geruchsbindenden Festpartikel sind in dieser Schutzschicht verteilt.

In Figur 2 ist zwischen der Kapselhülle 2 und der Schutzschicht 3 noch eine Sperrschicht 4 z.B. aus einem konventionellen Lack angeordnet.

Bei einem Ausführungsbeispiel werden mit einem Knoblauchpräparat gefüllte Weichgelatinekapseln mit und ohne Sperrschicht in einem Brucks-Dragierkessel mit einer Suspension behandelt, welche folgende Zusammensetzung aufweist: Saccharose (Zucker), Talcum, Bolus alba, Calciumcarbonat, Titandioxid, Gummi arabicum und Polyehtylenglykol. Es wurde dabei eine stabile und gut haftende Schutzschicht erzielt.

## Patentansprüche

1. Kapsel mit einer vorzugsweise flüssigen Kapselfüllung (1) und mit einer Kapselhülle (2) aus einer wasserlöslichen Substanz, vorzugsweise aus Biopolymeren, wobei die Aussenseite der Kapselhülle (2) mit einer mechanisch stabilen Schutzschicht (3) aus einer wasserlöslichen Substanz versehen ist, dadurch gekennzeichnet, dass die Schutzschicht (3) geruchsbindende Festpartikel aufweist.

2. Kapsel nach Anspruch 1, dadurch gekennzeichnet, dass die geruchsbindenden Festpartikel aus Aktivkohle oder Bentonit oder Kieselerde oder Kieselgur oder Kombinationen dieser Substanzen bestehen.

3. Kapsel nach einem der Ansprüche 1 oder 2, dadurch gekennzeichnet, dass die Schutzschicht (3) eine Schichtdicke von wenigstens 200 µm aufweist.

4. Kapsel nach einem der Ansprüche 1 bis 3, dadurch gekennzeichnet, dass die Schutzschicht (3) als Bestandteil eine Zuckerart oder Calciumcarbonat oder Calciumstearat oder Gummi Arabicum oder Stärke oder Talcum oder Kombinationen dieser Substanzen enthält.

5. Kapsel nach einem der Ansprüche 1 bis 4, dadurch gekennzeichnet, dass die Schutzschicht (3) als Bestandteil färbende und/oder aromatisierende Komponenten aufweist.

6. Kapsel nach einem der Ansprüche 1 bis 5, dadurch gekennzeichnet, dass zwischen der Kapselhülle und der Schutzschicht eine Sperrschicht aus einem Lack oder aus einem Harz angeordnet ist.

7. Verfahren zur Herstellung von Kapseln nach einem der Ansprüche 1 bis 6, dadurch gekennzeichnet, dass die Bestandteile der Schutzschicht (3) in einer wässrigen Suspension oder Lösung aufbereitet werden, und dass die Kapselhüllen bei gleichzeitiger oder nachfolgender Trocknung so lange beschichtet werden, bis die gewünschte Schichtdicke erreicht ist.

8. Verfahren nach Anspruch 7, dadurch gekennzeichnet, dass die Kapselhüllen vor dem Auftragen der Schutzschicht mit einem Lack oder mit einem Harz beschichtet werden.

9. Verfahren nach Anspruch 7, dadurch gekennzeichnet, dass die Kapselhüllen vor dem Auftragen der Schutzschicht durch Behandlung mit Formaldehyd gehärtet werden.

## Claims

1. Capsule with preferably a liquid capsule filling (1) and with a capsule envelope (2) of a water soluble material, preferably of biopolymers, whereby the outside of the capsule envelope (2) is provided with a mechanically stable protective layer (3) of a water soluble material, characterized in that the protective layer (3) features deodorizing solid particles.

2. Capsule according to claim 1, characterized in that the deodorizing solid particles comprise active charcoal or bentonite or siliceous earth or diamotaceous earth or combinations of these substances.

3. Capsule according to one of the claims 1 or 2, characterized in that the protective layer (3) has a thickness of at least 200 um.

4. Capsule according to one of the claims 1 to 3, characterized in that the protective layer (3) contains as a constituent a sugar type or calcium carbonate or calcium stearate or gum acacia or starch or talc or combinations of these substances.

5. Capsule according to one of the claims 1 to 4, characterized in that the protective layer (3) features as a constituent colouring and/or aromatizing components.

6. Capsule according to one of the claims 1 to 5, characterized in that a barrier of a varnish or a resin is arranged between the capsule envelope and the protective layer.

EP 0 317 510 B1

7. Method of manufacturing capsules according to one of the claims 1 to 6, characterized in that the constituents of the protective layer (3) are prepared in aquatic suspension or solution and that the capsule envelopes are coated, with simultaneous or subsequent drying, for such a time until the desired layer thickness is reached.

8. Method according to claim 7, characterized in that the capsule envelopes are coated with a varnish or a resin before application of the protective layer.

9. Method according to claim 7, characterized in that the capsule envelopes are hardened through treatment with formaldehyde before application of the protective layer.

**Revendications**

1. Capsule comportant un contenu de capsule (1) de préférence fluide et une enveloppe de capsule (2) en une substance soluble dans l'eau, de préférence en biopolymères, la face extérieure de l'enveloppe de capsule (2) étant munie d'une couche de protection (3) stable mécaniquement et constituée d'une substance soluble dans l'eau, caractérisée en ce que la couche de protection (3) présente des particules solides fixant les odeurs.

2. Capsule selon la revendication 1, caractérisée en ce que les particules solides fixant les odeurs sont constituées de charbon actif ou de bentonite ou de terre siliceuse ou de diatomite ou de combinaisons de ces substances.

3. Capsule selon l'une des revendications 1 ou 2, caractérisée en ce que la couche de protection (3) présente une épaisseur de couche d'au moins 200 μm.

4. Capsule selon l'une des revendications 1 à 3, caractérisée en ce que la couche de protection (3) contient, comme constituant, un sucre ou du carbonate de calcium ou du stéarate de calcium ou de la gomme arabique ou de l'amidon ou du talc ou des combinaisons de ces substances.

5. Capsule selon l'une des revendications 1 à 4, caractérisée en ce que la couche de protection (3) présente,comme constituant, des comporants colorants et/ou aromatisants.

6. Capsule selon l'une des revendications 1 à 5, caractérisée en ce qu'entre l'enveloppe de capsule et la couche de protection eat disposée une couche de barrage constituée d'une laque ou d'une résine.

7. Procédé de fabrication de capsules selon l'une des revendications 1 à 6, caractérisé en ce que l'on prépare les constituants de la couche de protection (3) dans une suspension ou une solution aqueuse, et en ce qu'on revêt les enveloppes de capsule jusqu'à atteindre l'épaisseur de couche désirée tout en les séchant simultanément ou ultérieurement.

8. Procédé selon la revendication 7, caractérisé en ce qu'on revêt les enveloppes de capsule d'une laque ou d'une résine avant d'appliquer la couche de protection.

9. Procédé selon la revendication 7, caractérisé en ce qu'on durcit les enveloppes de capsule par un traitement avec de l'aldéhyde formique avant d'appliquer la couche de protection.

Fig. 1

Fig. 2